(19) Europäisches Patentamt
European Patent Office
Office européen des brevets

(11) EP 0 620 217 B1

(12) EUROPÄISCHE PATENTSCHRIFT

(45) Veröffentlichungstag und Bekanntmachung des Hinweises auf die Patenterteilung:
**29.05.1996 Patentblatt 1996/22**

(51) Int Cl.[6]: **C07D 239/26**, C07D 239/36, C07D 239/40, C07D 239/42

(21) Anmeldenummer: **94103290.6**

(22) Anmeldetag: **04.03.1994**

(54) **Verfahren zur Herstellung von 4-Methylpyrimidinen**

Process for the preparation of 4-methyl-pyrimidines

Procédé pour la préparation des dérivés de 4-methylpyrimidine

(84) Benannte Vertragsstaaten:
**BE CH DE FR GB IT LI NL**

(30) Priorität: **13.03.1993 DE 4308073**

(43) Veröffentlichungstag der Anmeldung:
**19.10.1994 Patentblatt 1994/42**

(73) Patentinhaber: **BASF Aktiengesellschaft**
**D-67063 Ludwigshafen (DE)**

(72) Erfinder:
- **Rittinger, Stefan, Dr.**
  **D-67059 Ludwigshafen (DE)**
- **Rieber, Norbert, Dr.**
  **D-68259 Mannheim (DE)**

(56) Entgegenhaltungen:
- **J.HETEROCYCLIC CHEM. Bd. 22 , November 1985 Seiten 1723 - 1726 C.A.LIPINSKI ET AL '2-Amino and 2-Guanidino-4-thiazolylpyrimidines'**
- **J.HETEROCYCLIC CHEM. Bd. 27, Nr. 2 , 1990 Seiten 295 - 305 P.SCHENONE ET AL 'Reaction of 2-Dimethylaminomethylene-1,3-diones with dinucleophiles'**
- **CHEM. BERICHTE Bd. 90 , 1957 Seiten 942 - 952 H.BREDERECK ET AL 'Formamid-Reaktionen, VIII'**
- **CHEMICAL ABSTRACTS, vol. 73, no. 13, 28. September 1970, Columbus, Ohio, US; abstract no. 66528s, 'Cyclisation of diacetylene and its homologs with guanidine and its derivatives' Seite 341 ;**
- **CHEMICAL ABSTRACTS, vol. 84, no. 17, 26. April 1976, Columbus, Ohio, US; abstract no. 121763u, 'Pyrimidines. LII' Seite 536 ;**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von 4-Methylpyrimidinen durch Umsetzung von 1-Aminovinylmethylketonen mit Säureamiden.

Aus Chem. Ber. 90, 942 bis 952 (1957) und in J. Heterocycl. Chem. 22, 1723-26 (1985) ist ein Verfahren zur Herstellung von Pyrimidinen ausgehend von 1,3-Dicarbonylverbindungen bekannt, die sämtlich entweder nicht im technischen Maßstab verfügbar oder nicht preiswert sind.

In Zh. Org. Khim. 6, 1347 bis 1348 (1970) wird die Umsetzung von Diacetylen mit Guanidinderivaten zu entsprechend substituierten 4-Methylpyrimidinen beschrieben. Die angegebenen Ausbeuten von 20 % und die Notwendigkeit des Arbeitens mit reinem Diacetylen stehen jedoch einer technischen Anwendung entgegen.

Der vorliegenden Erfindung lag daher die Aufgabe zugrunde, den zuvor genannten Nachteilen abzuhelfen.

Demgemäß wurde ein neues und verbessertes Verfahren zur Herstellung von 4-Methylpyrimidinen der allgemeinen Formel I

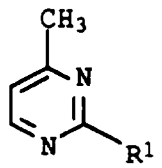

(I),

in der $R^1$ $C_1$- bis $C_{20}$-Alkyl, $C_3$- bis $C_8$-Cycloalkyl, Aryl, $C_7$- bis $C_{12}$-Phenalkyl, $C_7$- bis $C_{12}$-Alkylphenyl, $NH_2$, NHCN, OH und SH bedeutet, gefunden, welches dadurch gekennzeichnet ist, daß man 1-Aminovinylmethylketone der allgemeinen Formel II

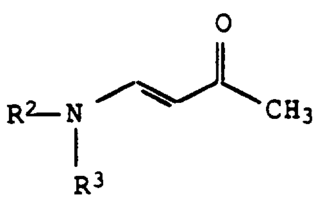

(II),

in der $R^2$ und $R^3$ $C_1$- bis $C_{20}$-Alkyl, $C_3$- bis $C_8$-Cycloalkyl, Aryl, $C_7$- bis $C_{12}$-Phenylalkyl, $C_7$- bis $C_{12}$-Alkylphenyl, $C_1$- bis $C_{20}$-Hydroxyalkyl oder gemeinsam eine gegebenenfalls ein- bis vierfach durch $C_1$- bis $C_4$-Alkyl substituierte gegebenenfalls durch Sauerstoff, Stickstoff oder Schwefel unterbrochene $C_2$- bis $C_7$-Alkylenkette
bedeuten, mit Carbonsäureamiden oder Amidinen oder deren Salze der allgemeinen Formel III

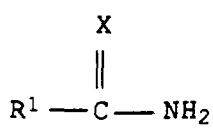

(III),

in der $R^1$ die obengenannten Bedeutungen hat und X für Sauerstoff oder NH steht, bei Temperaturen von 20 bis 200°C und Drücken von 0,01 bis 50 bar umsetzt sowie die Herstellung der 1-Aminovinylmethylketone II aus Diacetylen und sekundären Aminen, indem man

a) aus dem bei der Acetylenerzeugung resultierenden Spaltgas ein Teilstrom, der Diacetylen der Formel IV

$$H\text{-}C{\equiv}C\text{-}C{\equiv}C\text{-}H \qquad (IV),$$

enthält, durch Absorption abtrennt und

b) diesen Teilstrom mit sekundären Aminen der allgemeinen Formel V

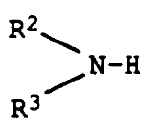

(V),

in der $R^2$ und $R^3$ die obengenannten Bedeutungen haben, und Wasser gegebenenfalls in einem Verdünnungsmittel bei Temperaturen von 0 bis 150°C und Drücken von 0,01 bis 5 bar umsetzt.

Das erfindungsgemäße Verfahren läßt sich wie folgt durchführen:

Die 1-Aminovinylmethylketone II und die Carbonsäureamide III können gegebenenfalls in einem inerten Lösungsmittel vorgelegt und die Carbonsäureamide III unter Temperaturkontrolle zugegeben werden.

Die Umsetzung kann gegebenenfalls in Gegenwart 0,001 bis 1000 Gew.-%, bevorzugt 0,005 bis 500 Gew.-%, besonders bevorzugt 0,01 bis 200 Gew.-% einer Säure oder einer Base als Katalysator bei Temperaturen von 20 bis 200°C, bevorzugt 40 bis 180°C, besonders bevorzugt 50 bis 160°C und Drücken von 0,01 bis 50 bar, bevorzugt 0,1 bis 5 bar, besonders bevorzugt bei Atmosphärendruck (Normaldruck) sowohl diskontinuierlich als auch bevorzugt kontinuierlich in der Gasphase oder bevorzugt in der Flüssigphase durchgeführt werden. Vorteilhaft kann man das entstehende Reaktionswasser durch an sich bekannte Schleppmittel auskreisen.

Als inerte Lösungsmittel eignen sich Formamide, z.B. Dialkylformamide wie Dimethylformamid, Lactame wie N-Methylpyrrolidon, Alkohole, z.B. $C_1$- bis $C_{20}$-Alkanole wie Methanol und Ethanol, aromatische Kohlenwasserstoffe, z. B. $C_7$- bis $C_{20}$-Alkylaryle wie Benzol, Toluol, o-Xylol, m-Xylol, p-Xylol, Xylol-Isomerengemische, bevorzugt Aromatengemische, die üblicherweise bei der Spaltung von Kohlenwasserstoffen entstehenden Aromatengemische (Rückstandsöle), oder Glykolether z.B. wie Ethylenglykol-diethylether.

Als Säuren eignen sich beispielsweise Mineralsäuren wie Salzsäure, Schwefelsäure, Salpetersäure und Phosphorsäure, Sulfonsäuren wie Methansulfonsäure und p-Toluolsulfonsäure.

Als Basen eignen sich anorganische Basen, beispielsweise Hydroxide wie Natriumhydroxid, Kaliumhydroxid und Calciumhydroxid oder organische Basen beispielsweise Ammoniak, primäre, sekundäre und tertiäre Amine, Alkoholate z.B. $C_1$- bis $C_8$-Alkali- und Erdalkalialkoholate wie Natriummethanolat, Pyridin und Imidazole.

Das vorliegende Verfahren eignet sich im besonderen zur Verwertung von diacetylenhaltigen Teilströmen. Das - bei der Zerlegung von Spaltgasen aus der Spaltung von Kohlenwasserstoffen unter den Bedingungen der Acetylensynthese als verdünnter Teilstrom - anfallende Gemisch höherer Acetylene kann ohne weitere physikalische oder chemische Behandlung unmittelbar mit wäßrigen Lösungen von sekundären Aminen bei Temperaturen von 0 bis 150°C, bevorzugt 30 bis 130°C, besonders bevorzugt 40 bis 120°C und Drücken von 0,01 bis 5 bar, bevorzugt 0,1 bis 2,5 bar, besonders bevorzugt 0,5 bis 1,5 bar, in der Regel bei Normaldruck (Atmosphärendruck), umgesetzt werden.

Die technischen Varianten zur Erzeugung und Aufbereitung der erfindungsgemäß verwendeten Spaltgase ("Crack-Gase") ist z.B. aus Ullmans Encyclopedia of Industrial Chemistry, V. Edition, A1, 1985, Seite 97 bis 145, insbesondere Seite 111, Fig. 13 bekannt, wobei Kohlenwasserstoffe (z. B. Erdgas oder auch höhersiedende Fraktionen) bei den für die Acetylenentstehung notwendigen, hohen Temperaturen gespalten und die resultierenden Produkte unmittelbar nach der Reaktionszone (Spaltzone) durch Eindüsen einer Flüssigkeit (z. B. Wasser oder Öl) rasch abgekühlt ("Quench") werden. Die Zusammensetzung der Spaltgase ist abhängig von den zur Spaltung eingesetzten Ausgangsstoffen, sowie den Spaltungsbedingungen.

Die erfindungsgemäß verwendeten Teilströme fallen bei der Zerlegung des Spaltgases durch eine Serie von physikalischen Trennvorgängen, bevorzugt durch Absorptions-/Desorptionsprozesse in einer Serie von Wasch- und Stripkreisläufen, in mehreren Teilströmen an. Die verwendbaren, höhere Acetylene enthaltenden Teilströme haben gegenüber dem Spaltgas deutlich höhere Konzentrationen an Diacetylen.

Eine weitere Aufbereitung des Einsatzstromes gemäß DE-A-21 57 537 ist nicht notwendig. Sowohl flüssige Einsatzströme als auch gasförmige, durch Strippen (Desorption) der Waschlösungen erhältliche Teilströme zeichnen sich gegenüber dem Spaltgas durch ein für die gewünschte Umsetzung zu Aminovinylmethylketon günstigeres Verteilungsprofil der höheren Acetylene aus. Es sind nur noch unwesentliche Mengen anderer, höherer Acetylene (Pentadiin, Hexadiin, etc.) enthalten, da diese vorher abgeschieden werden. Dies führt zu einer hohen Reinheit des erhaltenen Produktes. Die Abtrennung der höheren Acetylene aus dem Spaltgas erfolgt in bevorzugter Weise so, daß die höheren Acetylene in einer Vorwäsche des das Gesamtacetylen enthaltenden Spaltgases mit einer Flüssigkeit absorbiert werden. Diese mit Diacetylen angereicherte Waschlösung kann dann noch in einem Vakuumstripper entgast werden, wobei die Gasfraktion nochmals vor oder unmittelbar nach einer eventuellen Kompression durch Zumischen eines Inertgasanteils stabilisiert wird.

Diacetylenhaltige Kohlenwasserstoffkondensate (sog. BTX-Fraktion) aus diesem Gasstrom eignen sich gleichermaßen vorteilhaft für die beschriebene Umsetzung von Diacetylen zu 1-Aminovinylmethylketon. Dies bewirkt für diese Vorgehensweise den zusätzlichen Vorteil, daß nach Abreaktion des Diacetylens destillativ die von Diacetylen befreite BTX-Fraktion erhalten werden kann, welche beispielsweise wieder als Einsatzstoff zur Crackung verwendet werden kann.

Als Absorptionsmittel in der Spaltgasaufbereitung eignen sich hochsiedende Kohlenwasserstoffe wie Aromatengemische, bevorzugt Rückstandsöle, N-Alkyllactame mit $C_1$- bis $C_3$-Alkyl, bevorzugt N-Methylpyrrolidon, $C_1$- bis $C_5$-Alkohole, bevorzugt Methanol, Säureamide, bevorzugt Dimethylformamid, alkylierte cyclische Harnstoffe, bevorzugt Dimethylpropylenharnstoff, primäre, sekundäre und tertiäre $C_1$- bis $C_6$-Amine und Ammoniak.

Zur Verdünnung der aus dem Absorbens entfernten diacetylenhaltigen Gase eignen sich Inertgase wie Kohlenwasserstoffe, Kohlenmonoxid, Synthesegas, Armgas, Stickstoff oder Gemische dieser Gase, bevorzugt Erdgas und/oder Acetylen.

Das inertisierte Gasgemisch enthält in der Regel bevorzugt 55 bis 85 Vol-% Inertgas, 1 bis 30 Vol-% Diacetylen und 10 bis 20 Vol-% andere Bestandteile wie Acetylen, Vinylacetylen und Benzol. Der Anteil des Diacetylens im inertisierten Gasgemisch beträgt in der Regel 1 bis 30 Vol-%, bevorzugt 5 bis 20 Vol-%. Die Obergrenze wird durch die Grenze des explosiven Selbstzerfalls im Inertgas bestimmt.

Typische gasförmige, höhere Acetylene enthaltende Teilströme haben folgende Zusammensetzung:

- 58 Vol-% Methan, 18 Vol-% Diacetylen, 5 Vol-% Stickstoff, 4 Vol-% Acetylen, 4,5 Vol-% Vinylacetylen, 4 Vol-% Benzol, 2 Vol-% Ethan, 2 Vol-% Cyclopentadien, 2,5 Vol-% Restbestandteile.

Typische flüssige, höhere Acetylene enthaltende Teilströme haben folgende Zusammensetzung:

- 32 Vol-% Benzol, 28 Vol-% Toluol, 17 Vol-% Xylol (BTX), 8 Vol-% Styrol, 6 Vol-% Diacetylen,
- Methanol (89 Vol-%), Acetylen (0,6 Vol-%), Diacetylen (2,2 Vol-%), Vinylacetylen (0,4 Vol-%), Cyclopentadien (1 Vol-%), Benzol (1,8 Vol-%) und Toluol (1,3 Vol-%).

Die Umsetzung des Teilstroms mit wäßrigen sekundären Aminen kann sowohl diskontinuierlich als auch kontinuierlich gestaltet werden. Bei Nutzung eines gasförmigen Teilstroms sind Verfahren, die eine gute Gasverteilung in Flüssigkeiten erzielen, z.B. die Verwendung von Apparateteilen wie Begasungsring, Lochboden, verdichtende Begasungseinrichtungen, Sprühreaktor oder Absorberturm vorteilhaft.

Nicht vollständig umgesetztes Amin kann destillativ vom Produkt oder dem bei unmittelbarer Weiterverarbeitung erhältlichen Folgeprodukt abgetrennt und in die Synthesestufe a) zurückgeführt werden.

Das Gasgemisch aus der Spaltgasaufbereitung hat gewöhnlich gegenüber der Umgebung leicht erhöhte Betriebstemperatur. Die Vorabscheidung von leicht kondensierbaren Gasbestandteilen in Abscheidebehältern in der Reaktorzuleitung bei Umgebungstemperatur verbessert die Reinheit des Rohproduktes.

Die Substituenten $R^1$, $R^2$, $R^3$ und X in den Verbindungen I, II, III und V haben folgende Bedeutungen:

$R^1$, $R^2$ und $R^3$

- unabhängig voneinander
- $C_1$- bis $C_{20}$-Alkyl, bevorzugt $C_1$- bis $C_8$-Alkyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl,
- $C_3$- bis $C_8$-Cycloalkyl, bevorzugt $C_5$- bis $C_8$-Cycloalkyl wie Cyclopentyl, Cyclohexyl, Cycloheptyl und Cyclooctyl, besonders bevorzugt Cyclopentyl und Cyclohexyl,
- Aryl wie Phenyl, 1-Naphthyl und 2-Naphthyl, bevorzugt Phenyl, 1-Naphthyl und 2-Naphthyl, besonders bevorzugt Phenyl,
- $C_7$- bis $C_{12}$-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,5-Dimethylphenyl und 2,3,4-Trimethylphenyl,
- $C_7$- bis $C_{12}$-Phenylalkyl wie Benzyl, 1-Phenethyl, 2-Phenethyl, 1-Phenyl-propyl, 2-Phenyl-propyl, 1-Phenylbutyl, 2-Phenylbutyl, 3-Phenyl-butyl, bevorzugt Benzyl, 1-Phenethyl und 2-Phenethyl,

zusätzlich

$R^1$

- $NH_2$, NHCN, OH und SH.

$R^2$, $R^3$

- $C_1$- bis $C_{20}$-Hydroxyalkyl, bevorzugt $C_2$- bis $C_8$-Hydroxyalkyl, besonders bevorzugt C2- bis C4-hydroxyalkyl wie 2-Hydroxyethyl und 3-Hydroxypropyl,

$R^2$ und $R^3$

- gemeinsam

- eine gegebenenfalls durch ein bis vier $C_1$- bis $C_4$-Alkylreste substituierte gegebenenfalls durch Sauerstoff, Stickstoff oder Schwefel unterbrochene $C_2$- bis $C_7$-Alkylenkette wie $-(CH_2)_2-$, $-(CH_2)_3-$, $-(CH_2)_4-$, $-(CH_2)_5-$, $-(CH_2)_6-$, $-(CH_2)_7-$, $-(CHCH_3)-$, $-(CH_2)_2-O-(CH_2)_2-$, $-(CH_2)_2-N-(CH_2)_2-$ und $-(CH_2)_2-S-(CH_2)_2-$,

X

- Sauerstoff oder NH.

Die 4-Methylpyrimidine I sind Ausgangsprodukte zur Herstellung biologisch aktiver Wirksubstanzen (DE-A-816 700; US-A-2 725 384; DE-A-871 303; US-A-2 688 015, US-A-2 690 439 und US-A-2 690 466;

WO-A-92/10 490).

Beispiele

In den folgenden Beispielen ist das inertisierte, diacetylenhaltige Gasgemisch mit HA-Gas bezeichnet. Das Diacetylen im HA-Gas bzw. im Abgas nach der Reaktion wurde gaschromatographisch auf einer gepackten Säule (20 % Reoplex 400 auf Chromosorb PAW) mit Trägergas $N_2$ (35 ml/min.) und FID-Detektion bestimmt. Die Konzentrationen sind in Vol-% angegeben.

Beispiel 1

In einem 500 ml-Rührbehälter wurden eine Mischung aus 67 g Morpholin und 33 g $H_2O$ vorgelegt und auf 80°C erhitzt. 15 l/h eines Teilstroms des HA-Abgases der Acetylenproduktion wurden durch ein Einleitungsrohr mit Glasfritte während 12 Betriebsstunden durch die Reaktionslösung hindurchgeleitet. Die Diacetylenkonzentration des HA-Gases am Reaktoreingang schwankte von 4 bis 9 %. Bei einer durchschnittlichen Abreicherung des Diacetylens von über 90 % resultierte 130 g dunkelgefärbte Reaktionslösung mit einem Restmorpholingehalt von 18 % und einem Gehalt von 60 % 1-Morpholinovinylmethylketon. Nach Aufsetzen einer Destillationsbrücke wurden bei ca. 200 mbar/75°C ein Gemisch aus Wasser und Morpholin abestilliert. Restliches Amin wurde durch Verminderung des Drucks auf 20 mbar entfernt. Der schwarzbraun gefärbte Rückstand bestand zu 90 % (85 g) aus 1-Morpholinovinylmethylketon.

Beispiel 2

85 g Enaminketon aus Beispiel 1 (bei 90%iger Reinheit entsprechend 0,5 mol) wurden in einem 1 l-Rührkolben aus Glas mit 500 ml Xylol verdünnt. Der Lösung wurden 0,5 mol (90 g) Guanidiniumcarbonat zugesetzt und anschließend wurde unter Rühren am Wasserkreiser zum Rückfluß erhitzt. Nach 60 h war im GC kein Enaminketon mehr detektierbar. Die Auskreisung des Produktwassers war gleichfalls quantitativ (0.5 mol = 9 g). Der Reaktorinhalt wurde filtriert. Aus dem Filtrat wurden nach Abdestillieren des Lösungsmittels 45,2 g (75%) 2-Amino-4-methylpyrimidin; Smp.: 160 bis 162°C in Form farbloser Kristalle erhalten.

Beispiel 3

In einer 500 ml-Rührapparatur wurden in 200 ml Ethanol 5,4 g Natriummethanolat, 9,5 g Acetamidiniumchlorid und 17,2 g 90%iges Enaminketon aus Beispiel 1 eingetragen (je 0,1 mol). Nach 3 stündigem Kochen am Rückfluß hatte sich das Enaminketon völlig umgesetzt und die anfangs braune Suspension fast völlig entfärbt (blaßgelb). Die Reaktionslösung zeichnete sich durch einen unangenehmen, pyridinähnlichen Geruch aus. 2,4-Dimethylpyrimidin konnte gaschromatographisch durch Zuspritzen einer authentischen Probe identifiziert werden. Aus dem Gehalt in der Reaktionslösung (6 Flächen-%) ergab sich eine Ausbeute 80 %.

**Patentansprüche**

**1.** Verfahren zur Herstellung von 4-Methylpyrimidinen der allgemeinen Formel I

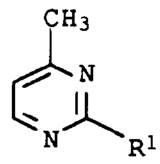

(I),

in der $R^1$ $C_1$- bis $C_{20}$-Alkyl, $C_3$- bis $C_8$-Cycloalkyl, Aryl, $C_7$- bis $C_{12}$-Phenalkyl, $C_7$- bis $C_{12}$-Alkylphenyl, $NH_2$, NHCN, OH und SH

bedeuten, dadurch gekennzeichnet, daß man 1-Aminovinylmethylketone der allgemeinen Formel II

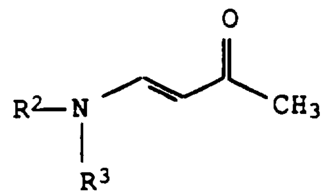

(II),

in der $R^2$ und $R^3$ $C_1$- bis $C_{20}$-Alkyl, $C_3$- bis $C_8$-Cycloalkyl, Aryl, $C_7$- bis $C_{12}$-Phenylalkyl, $C_7$- bis $C_{12}$-Alkylphenyl, $C_1$- bis $C_{20}$-Hydroxyalkyl oder gemeinsam eine gegebenenfalls ein- bis vierfach durch $C_1$- bis $C_4$-Alkyl substituierte gegebenenfalls durch Sauerstoff, Stickstoff oder Schwefel unterbrochene $C_2$- bis $C_7$-Alkylenkette

bedeuten, mit Carbonsäureamiden oder Amidinen oder deren Salze der allgemeinen Formel III

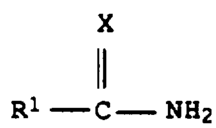

(III),

in der $R^1$ die obengenannten Bedeutungen hat und X für Sauerstoff oder NH steht, bei Temperaturen von 20 bis 200°C und Drücken von 0,01 bis 50 bar umsetzt.

**2.** Verfahren zur Herstellung von 4-Methylpyrimidinen I nach Anspruch 1, dadurch gekennzeichnet, daß man die 1-Aminovinylmethylketone II aus Diacetylen und sekundären Aminen erhält, indem man

a) aus dem bei der Acetylenerzeugung resultierenden Spaltgas ein Teilstrom, der Diacetylen der Formel IV

$$H\text{-}C{\equiv}C\text{-}C{\equiv}C\text{-}H \qquad \text{(IV)},$$

enthält, durch Absorption abtrennt und

b) diesen Teilstrom mit sekundären Aminen der allgemeinen Formel

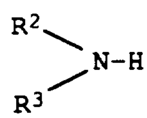

(V),

in der $R^2$ und $R^3$ die obengenannten Bedeutungen haben, und Wasser gegebenenfalls in einem Verdünnungsmittel bei Temperaturen von 0 bis 150°C und Drücken von 0,01 bis 5 bar umsetzt.

**3.** Verfahren zur Herstellung von 4-Methylpyrimidinen I nach Anspruch 1, dadurch gekennzeichnet, daß man die Umsetzung bei Temperaturen von 40 bis 180°C durchführt.

## Claims

**1.** A process for the preparation of a 4-methylpyrimidine of the formula I

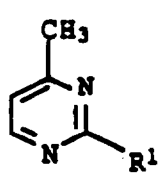

(I),

where $R^1$ is $C_1$-$C_{20}$-alkyl, $C_3$-$C_8$-cycloalkyl, aryl, $C_7$-$C_{12}$-phenalkyl, $C_7$-$C_{12}$-alkylphenyl, $NH_2$, NHCN, OH or SH, wherein a 1-aminovinyl methyl ketone of the formula II

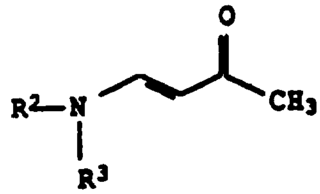

(II),

where $R^2$ and $R^3$ are each $C_1$-$C_{20}$-alkyl, $C_3$-$C_8$-cycloalkyl, aryl, $C_7$-$C_{12}$-phenylalkyl, $C_7$-$C_{12}$-alkylphenyl or $C_1$-$C_{20}$-hydroxyalkyl or together are a $C_2$-$C_7$-alkylene chain which is unsubstituted or monosubstituted to tetrasubstituted by $C_1$-$C_4$-alkyl and may be interrupted by oxygen, nitrogen or sulfur,
is reacted with a carboxamide or an amidine or a salt thereof of the formula III

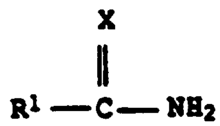

(III),

where $R^1$ has the abovementioned meanings and X is oxygen or NH, at from 20 to 200°C and from 0.01 to 50 bar.

**2.** A process for the preparation of a 4-methylpyrimidine I as claimed in claim 1, wherein the 1-aminovinyl methyl ketone II is obtained from diacetylene and a secondary amine by

a) separating a part-stream which contains diacetylene of the formula IV

$$H-C\equiv C-C\equiv C-H \quad (IV),$$

by absorption from the crack gas resulting from the production of acetylene and
b) reacting this part-stream with a secondary amine of the formula

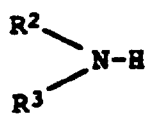

(V),

where $R^2$ and $R^3$ have the abovementioned meanings, and water, if required in a diluent, at from 0 to 150°C and from 0.01 to 5 bar.

**3.** A process for the preparation of a 4-methylpyrimidine I as claimed in claim 1, wherein the reaction is carried out at from 40 to 180°C.

## Revendications

**1.** Procédé de préparation de 4-méthylpyrimidines de la formule générale I

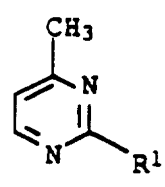

(I),

dans laquelle $R^1$ représente un radical alkyle en $C_1$ à $C_{20}$, cycloalkyle en $C_3$ à $C_8$, aryle, phénalkyle en $C_7$ à $C_{12}$, alkylphényle en $C_7$ à $C_{12}$, $NH_2$, NHCN, OH et SH
caractérisé en ce que l'on fait réagir des 1-aminovinylméthylcétones de la formule générale II

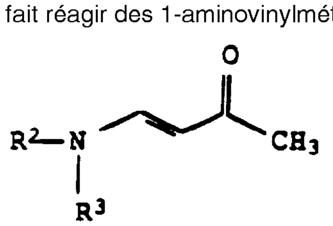

(II),

dans laquelle $R^2$ et $R^3$ représentent des radicaux alkyle en $C_1$ à $C_{20}$, cycloalkyle en $C_3$ à $C_8$, aryle, phénylalkyle en $C_7$ à $C_{12}$, alkylphényle en $C_7$ à $C_{12}$, hydroxyalkyle en $C_1$ à $C_{20}$, ou forment en commun, une chaîne alkylène en $C_2$ à $C_7$, éventuellement une à quatre fois substituée par des radicaux alkyle en $C_1$ à $C_4$, éventuellement interrompue par de l'oxygène, de l'azote ou du soufre,
avec des amides d'acides carboxyliques, ou des amidines, ou leurs sels, de la formule générale III

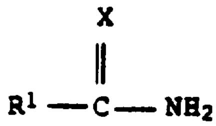

(III),

dans laquelle $R^1$ possède les mêmes significations que celles qui lui ont été attribuées ci-dessus et X représente un atome d'oxygène ou le radical NH, à des températures de 20 à 200°C et sous des pressions de 0,01 à 50 bars.

2. Procédé de préparation de 4-méthylpyrimidines I suivant la revendication 1, caractérisé en ce que l'on obtient les 1-aminovinylméthylcétones II à partir du diacétylène et d'amines secondaires, pour autant que

   a) on sépare par absorption d'avec le gaz de scission résultant de l'obtention de l'acétylène, un courant partiel qui contient le diacétylène de la formule IV

   $$H\text{-}C{\equiv}C\text{-}C{\equiv}C\text{-}H \qquad (IV),$$

   et

   b) on fasse réagir ce courant partiel avec des amines secondaires de la formule générale

   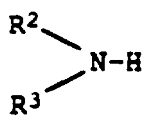

   (V),

   dans laquelle $R^2$ et $R^3$ possèdent les significations qui leur ont été précédemment attribuées et de l'eau, éventuellement dans un diluant, à des températures de 0 à 150°C et sous des pressions de 0,01 à 5 bars.

3. Procédé de préparation de 4-méthylpyrimidines I suivant la revendication 1, caractérisé en ce que l'on entreprend la réaction à des températures de 40 à 180°C.